(19) Europäisches Patentamt
European Patent Office
Office européen des brevets

(11) Veröffentlichungsnummer: **0 313 630 B1**

# (12) EUROPÄISCHE PATENTSCHRIFT

(45) Veröffentlichungstag der Patentschrift: **16.09.92**

(51) Int. Cl.⁵: **C07D 401/12**, A61K 31/50, A61K 31/505

(21) Anmeldenummer: **88904462.4**

(22) Anmeldetag: **14.05.88**

(86) Internationale Anmeldenummer:
**PCT/DE88/00289**

(87) Internationale Veröffentlichungsnummer:
**WO 88/08842 (17.11.88 88/25)**

## (54) SUBSTITUIERTE 2-ACYLPYRIDIN-ALPHA-(N)-HETARYLHYDRAZONE SOWIE DIESE ENTHALTENDE ARZNEIMITTEL.

(30) Priorität: **14.05.87 DE 3716131**

(43) Veröffentlichungstag der Anmeldung:
**03.05.89 Patentblatt 89/18**

(45) Bekanntmachung des Hinweises auf die Patenterteilung:
**16.09.92 Patentblatt 92/38**

(84) Benannte Vertragsstaaten:
**AT BE CH DE FR GB IT LI LU NL SE**

(56) Entgegenhaltungen:

**Il Farmaco, vol. XL, no. 9, September 1985, C. Pellerano et al.: "Sistemi chelanti tridentati N-N-N- quali potenziali agenti antitumorali", pages 645-654**

(73) Patentinhaber: **DEUTSCHES AUSSÄTZIGEN HILFSWERK E.V.**
**Dominikanerplatz 4**
**W-8700 Würzburg(DE)**

(72) Erfinder: **SCHAPER, Klaus-Jürgen**
**Parkallee 39c**
**W-2061 Borstel(DE)**
Erfinder: **SEYDEL, Joachim K.**
**Mühloh 2**
**W-2061 Borstel(DE)**

(74) Vertreter: **UEXKÜLL & STOLBERG Patentanwälte**
**Beselerstrasse 4**
**W-2000 Hamburg 52(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Chemical Abstracts, vol. 84, 1st March 1976, Columbus, Ohio, US; C.V. Bell et al.: "Proton NMR spectra of E-and Z-pyridine-2-carboxaldehyde 2'-pyridyl-hydrazone and related compounds", page 410, abstract no. 58 093h

J. Inorg. Nucl. Chem. vol. 43, 1981, Pergamon Press Ltd (GB); A.E. Mihkelson: "Reactions of Pd(II) with a series of potentially tridentate nitrogen ligands-I", pages 123-126, see page 123, column 2, line 46 - page 124, column 1, line 3

Chemical Abstracts, vol. 91, no. 21, 19 November 1979, Columbus, Ohio, US; C. Pellerano et al.: "Some hydraziono-quinoline derivatives: 2-Ouinolylhydrazones.VI." see page 645, abstract no. 175 157n

J. Protozool. vol. 29, no. 1, 1982, The Society of Protozoologists, A. Shapiro et al.: "I vivo and in vitro activity by diverse chelators against Trypanosoma brucei brucei", pages 85-90, see page 85, abstract

## Beschreibung

Die Erfindung betrifft substituierte 2-Acylpyridin-α-(N)-hetarylhydrazone, deren Herstellung, sowie deren Verwendung als Arzneimittel zur Behandlung von mikrobiellen und insbesondere mycobakteriellen Infekten sowie von Malaria und malignen Tumoren.

Die pharmazeutische Wirksamkeit heterocyclisch substituierter 2-Acylpyridine ist bekannt. So weisen die Verbindungen des Typs der 2-Acylpyridin-thiosemicarbazone pharmazeutische Wirksamkeit bei der Behandlung bakterieller und insbesondere mycobakterieller Erkrankungen wie Lepra und Tuberkulose auf. Sie sind ferner als Mittel zur Behandlung von Malaria und malignen Tumoren geeignet.

Nachteilig an diesen Verbindungen ist jedoch ihre hohe Toxizität. So wird beispielsweise von K.C.Agrawal et al. in Biochem. Pharmacol. 23, 2421 - 2429 (1974) über die schwere gastrointestinale Toxizität von 5-Hydroxy-pyridin-2-(carbox)aldehyd-thiosemicarbazon berichtet, einer Verbindung, die antileukämische Wirksamkeit aufweist. Auf die Appetitlosigkeit, Übelkeit und Erbrechen hervorrufende Wirkung der Thiosemicarbazone und deren Derivate allgemein weist u.a. L. Weinstein in Pharmacol. Basis Ther. 5. Auflage, 1201 bis 1223 (1975) hin.

Aufgabe der Erfindung ist es demgemäß, neue Verbindungen zu schaffen sowie pharmazeutische Wirkstoffe bereitzustellen, deren Wirkung derjenigen von Verbindungen des Typs der 2-Acylpyridin-thiosemicarbazone überlegen ist, bei gleichzeitig verringerter Toxizität.

Zur lösung dieser Aufgabe werden Arzneimittel mit einem Gehalt an neuen substituierten 2-Acylpyridin-α-(N)-hetarylhydrazone der allgemeinen Formel I

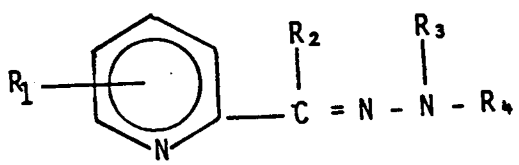

vorgeschlagen, in der $R_1$ Wasserstoff, Halogen, eine Alkylgruppe mit 1 bis 8 C-Atomen, eine Benzyloxy-Benzo-, Amino-, oder Acetaminogruppe,
$R_2$ Wasserstoff, eine Alkylgruppe mit 1 bis 8 C-Atomen, eine Amino- oder eine Phenylgruppe,
$R_3$ Wasserstoff oder eine Alkylgruppe mit 1 bis 3 C-Atomen und
$R_4$ ein Rest der allgemeinen Formeln II oder III

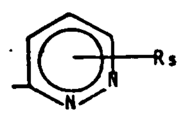

II

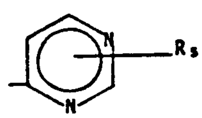

III

ist, in denen
$R_5$ Wasserstoff, Halogen, eine Alkyl- oder Alkoxygruppe mit 1 bis 4 C-Atomen, eine Benzogruppe oder eine Dialkylamino- oder substituierte Dialkylaminogruppe mit 1 bis 3 C-Atomen in den Alkylresten ist.

Bevorzugte Verbindungen sind in den Unteransprüchen angegeben.

Erfindungsgemäß zeigt es sich überraschenderweise, daß die Einführung der α-(N)-Hetarylgruppe an Stelle der Thioamidgruppe in Verbindungen vom Typ der 2-Acylpyridin-thiosemicarbazone bei Erhaltung bzw. Steigerung der pharmazeutischen Aktivität zu einer dramatischen Senkung der Toxizität gegenüber Säugetieren führt (vgl. Tabelle 1 für Pyridin-2'-pyridylhydrazon (PH22)).

Tabelle 1

| Akute Toxizität von PH22(I) Pyridin-2-Aldehyd-TSC(II) und Thiacetazon(III) bei Ratten und Mäusen | | |
|---|---|---|
| | | $LD_{50}$ (mg/kg Körpergewicht) |
| I<br>II<br>II | > 2000<br>30<br>40 | Ratte, subc.<br>Ratte, subc.<br>Maus i.p. |
| (Cancer Res. 25, 1454 (1965) | | |
| III | 1000 - 2000 | Maus, subc. |
| (J. Pharm. Pharmacol. 2, 764 (1950) | | |
| III | 950 | Maus, p.o. |
| (Comptes Rendus Soc.Biol. 1310 (1950) | | |

Die erfindungsgemäßen Verbindungen können hergestellt werden, indem man in an sich bekannter Weise die entsprechend substituierten Pyridinaldehyde oder -ketone mit den entsprechenden Hydrazinen umsetzt.

Die Wirksamkeit der erfindungsgemäßen Verbindungen als Wachstumsinhibitoren für insbesondere Mycobakterien, sowie für Malariaparasiten und Tumorzellen wurde nachgewiesen, indem die minimalen Hemmkonzentrationen (MHK) und die für eine halbmaximale Vermehrungsgeschwindigkeits-Hemmung ($I_{50}$) erforderlichen Konzentrationen bei Verwendung typischer Vertreter der erfindungsgemäßen Verbindungen ermittelt wurden.

Für die Hemmung des Wachstums verschiedener Mycobakterien-Stämme zeigte sich dabei eine um das 5- bis 200-Fache erhöhte Wirksamkeit der erfindungsgemäßen Verbindungen gegenüber handelsüblichen Präparaten (vgl. Tabelle 2).

Zusätzlich wurde ein synergistischer Effekt von Kombinationen der beanspruchten Verbindungen mit Hemmstoffen der Folatsynthase wie Sulfonen oder Sulfonamiden, der Dihydrofolsäurereduktase wie Methotrexat, Brodimoprim oder 2,4-Diamino-5-{4-[2-(4'-aminophenyl-4-sulfonylphenylamino)ethoxy]-3,5-dimethoxybenzyl}-pyrimidin, nachfolgend als K 107 bezeichnet und 2,4-Diamino-5-{4-[3-(4'-aminophenyl-4-sulfonylphenylamino)-propoxy]-3,5-dimethoxybenzyl}pyrimidin, nachfolgend als K 130 bezeichnet sowie mit Hemmstoffen der DNA-Synthese, wie beispielsweise 5-F-Uracil, Guanazol oder Desferal sowie mit Hemmstoffen der RNA-Polymerase (z.B. Rifampicin) gefunden. Dieser Effekt machte sich sowohl bei der Hemmung des Mycobakterienwachstums als auch bei der Hemmung des Wachstums von Malaria-Plasmodien bemerkbar (vgl. Tabellen 3 bis 7 sowie 9 und 10).

Die synergistische Wirksamkeit konnte noch gesteigert werden, wenn die erfindungsgemäßen Verbindungen gleichzeitig mit Hemmstoffen unterschiedlichen Typs kombiniert wurden. Die Bedeutung der erfindungsgemäßen Verbindungen und Arzneimittel liegt demgemäß u.a. auch in der Möglichkeit, partiell resistente Mycobakterien oder Malariaparasiten zu behandeln oder die Resistenzentwicklung bei der Monotherapie zu verringern. Hervorzuheben ist weiterhin die Wirksamkeit insbesondere in Kombinationen gegenüber mycobacteriellen Problemkeimen wie z.B. M. avium (Patientenstamm, Tab. 2).

Die erfindungsgemäßen Verbindungen weisen ferner in vitro an der humanen Mammatumor-Zellinie MDA-MB231 eine ausgeprägte cytostatische Wirksamkeit auf (Tab. 21).

Die erfindungsgemäßen Arzneimittel enthalten die erwähnten Wirkstoffe bzw. Wirkstoffkombinationen zusammen mit einem verträglichen pharmazeutischen Träger. Dieser Träger kann ein für die enterale, percutane und parenterale Verabreichung geeignetes organisches oder anorganisches Trägermaterial sein, wie z.B. Wasser, Gummi arabicum, Lactose, Stärke, Magnesiumstearat, Talg, pflanzliche Öle, Polyalkylenglykole oder Vaseline. Darüber hinaus können die Präparate weitere pharmazeutisch wirksame Stoffe enthalten, wie fiebersenkende Mittel, schmerzstillende Mittel oder entzündungshemmende Mittel. Die pharmazeutischen Präparate können oral, beispielsweise in Form von Tabletten, Kapseln, Pillen, Pulvern, Granulaten, Lösungen, Sirupen, Suspensionen oder, Elixieren verabreicht werden. Die Verabreichung kann aber auch parenteral, z.B. in Form von sterilen Lösungen, Suspensionen oder Emulsionen oder lokal in Form von Lösungen, Suspensionen, Salben, Pudern oder, Aerosolen erfolgen.

Ferner können die pharmazeutischen Präparate sterilisiert sein und/oder Bestandteile wie Konservierungsmittel, Stabilisatoren, Netzmittel, Emulgatoren, Salze und Puffersubstanzen enthalten.

Die Erfindung wird nachfolgend anhand von Beispielen erläutert.

**Beispiel 1**

Herstellung von Pyridin-2-aldehyd-1'-phthalazinylhydrazon (PH 22-1)

0,848 g 1-Hydrazinophthalazin.HCl wurden bei 50°C in 20 ml eines Gemisches aus Ethanol und Wasser (1:1) gelöst. Es wurden eine äquivalente Menge 1N NAOH und anschließend eine äquivalente Menge Pyridin-2-aldehyd (0,46 g) in 10 ml Ethanol/$H_2O$ hinzugegeben. Man ließ das Gemisch 20 Min. am Rückfluß kochen und anschließend abkühlen. Die Substanz wurde durch vorsichtiges Zugeben von Wasser aus der Lösung ausgefällt.

Es wurden 1,07 g kristalline gelbe Substanz mit einem Schmelzpunkt 193-194°C erhalten.

**Beispiel 2**

Herstellung von 2-Acetylpyridin-1'-phthalazinylhydrazon (PH 22-3)

0.5 g 2-Acetylpyridin wurden in 50 ml Wasser/Methylalkohol (1:1) gelöst und anschließend 0,82 g 1-Hydrazinophthalazin.HCl und 4,2 ml 1N NaOH zugegeben. Man ließ die Mischung 1,5 Stunden lang am Rückfluß kochen und anschließend abkühlen. Es fiel eine kristalline Substanz aus, die aus Ethanol/Wasser umkristallisiert wurde.

Die Ausbeute betrug 0,8 g einer gelben Substanz deren Schmelzpunkt bei 191 bis 195°C lag.

**Beispiel 3**

Herstellung von 5-Ethylpyridin-2-aldehyd-1'-phthalazinylhydrazon (PH 22-5).

20 g 5-Ethyl-2-methyl-pyridin wurden in 200 ml Eisessig gelöst, mit 35 ml Wasserstoffperoxid (30%-ig) versetzt und 6 Stunden lang bei einer Temperatur von 90 bis 100°C gerührt. Der Eisessig wurde abrotiert und der Rückstand mit Natriumcarbonat auf pH 8 eingestellt. Zur Entfernung der Ausgangssubstanz wurde die schwach alkalische Lösung mit Petrolbenzin extrahiert. Die wässrige Phase wurde anschließend mit Aktivkohle versetzt, kurz aufgekocht, heiß filtriert, abgekühlt und mit Dichlormethan extrahiert. Nach Eindampfen wurde eine Ausbeute von 16 g 5-Ethyl-2-methylpyridin-N-oxid erhalten.

Dieses wurde mit 5 ml Eisessig und 20 ml Essigsäureanhydrid 2 Stunden lang bei 90 bis 100°C gerührt. Die Reaktionslösung wurde auf Eis gegeben, neutralisiert, mit Dichlormethan extrahiert und die organische Phase anschließend einrotiert. Der Rückstand wurde in einer 5 Gew.% Natriumhydrogencarbonat enthaltenden wässrigen Lösung aufgenommen und mit Diethylether extrahiert. Nach Eindampfen des Extraktes wurden 14 g 2-Acetoxymethyl-5-ethylpyridin erhalten.

Dieses wurde in 100 ml 90%-igem Ethanol gelöst, mit 4,1 g NaOH versetzt und anschließend 1,5 Std. lang am Rückfluß gekocht. Die Lösung wurde anschließend mit Aktivkohle aufgekocht, das überschüssige NaOH mit 3 ml Eisessig neutralisiert und anschließend filtriert. Nach Einrotieren der Lösung wurde der Rückstand mit einer 5 gew.%-igen wässrigen Natriumhydrogencarbonatlösung aufgenommen, mit Diethylether extrahiert und die organische Phase einrotiert.

Es wurden 7 g 2-Hydroxymethyl-5-ethyl-pyridin erhalten.

Dieses wurde in 100 ml Trichlormethan gelöst, mit 8 g Mangandioxid versetzt und 2 Stunden lang am Rückfluß gekocht. Die Lösung wurde heiß filtriert und das Filtrat einrotiert.

Es wurden 4 g 5-Ethyl-pyridin-2-aldehyd erhalten.

2 g 5-Ethylpyridin-2-aldehyd wurden in 30 ml Wasser gelöst. Zu der Lösung wurden 2,2 g 1'-Hydrazinophthalazinhydrochlorid und 4,4 ml 1N NaOH in 20 ml Ethanol gegeben. Man ließ die Mischung 2 Stunden lang am Rückfluß kochen, setzte anschließend Aktivkohle zu und filtrierte in heißem Zustand. Die Substanz fiel zunächst als Öl aus, das nach 48 Stunden kristallisierte. Die Umkristallisation erfolgte aus Ethanol/Wasser.

Die Ausbeute betrug 0,81 g einer gelben Substanz, deren Schmelzpunkt bei 149-151°C lag.

**Beispiel 4**

Herstellung von 5-Benzyloxy-pyridin-2-aldehyd-1'-phthalazinylhydrazon (PH 22-6).

3,3 g Natrium wurden in 60 ml Methanol gelöst und mit 15 g 5-Hydroxy-2-methylpyridin in 230 ml DMSO eine Stunde lang bei einer Temperatur von 80 bis 90°C gerührt. Nach Abkühlen der Mischung

wurde das Methanol abrotiert. Anschließend wurden 23,5 g Benzylbromid zugegeben und 20 Stunden lang bei Raumtemperatur gerührt. Das Reaktionsgemisch wurde sodann mit 2N HCL angesäuert und mit 1500 ml Wasser versetzt. Die Mischung wurde anschließend mit Diethylether extrahiert und die Etherphase verworfen. Der pH-Wert der wässrigen Phase wurde anschließend auf etwa 8 eingestellt und die Lösung mit Ether extrahiert. Die organische Phase wurde eingedampft. Es wurden 24,1 g 5-Benzyloxy-2-methylpyridin erhalten.

Dieses wurde in 200 ml Eisessig gelöst, mit 12 ml Wasserstoffperoxid (30%-ig) versetzt und 2 Stunden lang am Rückfluß gekocht. Die Mischung wurde anschließend mit 40%-iger Natronlauge neutralisiert und 1000 ml Wasser hinzugegeben. Die Lösung wurde mit Dichlormethan extrahiert und die organische Phase eingedampft. Es wurden 25 g 5-Benzyloxy-2-methylpyridin-N-oxid erhalten.

Dieses wurde in 90 ml Essigsäureanhydrid gelöst und eine Stunde lang bei 100°C gerührt. Nach Zersetzen des überschüssigen Essigsäureanhydrids mit Eis wurde der pH-Wert der Lösung mit Natriumcarbonat auf 8 eingestellt und die Lösung anschließend mit Dichlormethan extrahiert. Es wurden 25 g 5-Benzyloxy-2-acetoxymethylpyridin erhalten.

Dieses wurde in 170 ml Methanol und 110 ml $H_2O$ gelöst, mit 12 g NaOH versetzt und anschließend 4 Stunden lang am Rückfluß gekocht. Die Lösung wurde dann auf einen pH-Wert von 1 eingestellt und mit Dichlormethan extrahiert. Die organische Phase wurde verworfen und die wässrige Phase mit Natriumcarbonat auf einen pH-Wert von 8 eingestellt und mit Dichlormethan extrahiert. Nach Eindampfen der organischen Phase wurden 15,4 g 5-Benzyloxy-2-hydroxymethylpyridin erhalten.

2,5 g dieser Substanz wurden in 100 ml Dichlormethan gelöst und mit 3 g aktiviertem Mangandioxid versetzt und 3 Stunden lang am Rückfluß gekocht. Die Lösung wurde anschließend heiß filtriert und eingedampft.

Es wurden 1,2 g 5-Benzyloxy-pyridin-2-aldehyd erhalten.

Dieser wurde in 45 ml Ethanol und 50 ml Wasser gelöst, 1,05 g 1-Hydrazinophthalazin.HCl und 5,3 ml 1N NaOH zugegeben und die Mischung eine Stunden lang am Rückfluß gekocht. Es fiel nach kurzer Zeit eine orange-gelbe Substanz aus, die jedoch mit 30 ml Ethanol wieder in Lösung gebracht wurde. Nach Zugeben von Aktivkohle wurde die Lösung heiß filtriert. Nach Abkühlen fiel eine kristalline Substanz aus, die aus Ethanol/Wasser umkristallisiert wurde.

Die Ausbeute betrug 0,6 g einer gelben Substanz deren Schmelzpunkt bei 162-164°C lag.

**Beispiel 5**

Herstellung von 4-Methyl-pyridin-2-aldehyd-1'-phthalazinylhydrazon (PH 22-8).

24,3 ml 2,4-Dimethylpyridin wurden in 80 ml Eisessig gelöst, die Lösung wurde mit 30 ml 30%-igem Wasserstoffperoxid versetzt und 25 Std. lang am Rückfluß gekocht. Anschließend wurde die Lösung mit gesättigter Natriumcarbonatlösung auf etwa pH 8 eingestellt, die Lösung mit Dichlormethan extrahiert und die organische Phase einrotiert. Der Rückstand wurde mit 90 ml Essigsäureanhydrid versetzt und 1,5 Stunden lang am Rückfluß gekocht. Anschließend wurde der pH-Wert der Lösung mit 40%-iger NaOH auf etwa 8 eingestellt und die Lösung mit Trichlormethan extrahiert.

Es wurden 30 g rohes 4-Methyl-2-acetoxymethylpyridin erhalten.

Dieses wurde in 150 ml Ethanol gelöst, mit 7,2 g NaOH versetzt und eine Stunde lang am Rückfluß gekocht. Anschließend wurde die Lösung filtriert, auf pH 2 eingestellt und mit Dichlormethan extrahiert. Die organische Phase wurde verworfen und die wässrige Phase mit NaOH auf pH 8 eingestellt und mit Petrolbenzin extrahiert. Die organische Phase wurde erneut verworfen und die wässrige Phase mit Dichlormethan extrahiert und der Extrakt einrotiert.

Es wurden 7 g 4-Methyl-2-hydroxymethylpyridin erhalten.

Dieses wurde in 150 ml Trichlormethan gelöst, mit 8 g aktiviertem Mangandioxid versetzt und 2 Stunden lang am Rückfluß gekocht. Die Lösung wurde heiß filtriert und das Filtrat zur Trockne einrotiert.

Es wurden 2,5 g 4-Methyl-pyridin-2-aldehyd erhalten.

1 g 4-Methylpyridin-2-aldehyd wurde zusammen mit 1,6 g 1-Hydrazinophthalazin.HCl in 50 ml 50%-igem Ethanol gelöst, die Lösung mit 4 ml 1N NaOH versetzt und 1 Stunde lang am Rückfluß gekocht. Anschließend wurde die Lösung mit wenig Wasser versetzt und abgekühlt. Es fiel eine kristalline Substanz aus, die aus Ethanol/Wasser umkristallisiert wurde.

Die Ausbeute betrug 0,65 g einer gelben Substanz, deren Schmelzpunkt bei 208 bis 213°C lag.

**Beispiel 6**

Herstellung von 5-Methyl-pyridin-2-aldehyd-1'-phthalazinylhydrazon (PH 22-16)

20 g 2,5-Dimethylpyridin wurden in 100 ml Eisessig gelöst und anschließend mit 35 ml 30%-igem $H_2O_2$ 5 Stunden lang bei 80 bis 90°C gerührt. Der überschüssige Eisessig wurde abrotiert und die Lösung mit 20%igem NaOH auf pH 8 eingestellt. Nach Extrahieren mit Petrolbenzin und Verwerfen der organischen Phase wurde die Lösung mit Dichlormethan extrahiert und die organische Phase einrotiert. Es wurden 12 g 2,5- Dimethylpyridin-N-oxid erhalten.

Dieses wurde mit 20 ml Acetanhydrid 10 Stunden lang bei 55 bis 75°C gerührt. Das Acetanhydrid wurde anschließend mit Wasser zersetzt und die gebildete Essigsäure mit 10 gew.%-iger $Na_2CO_3$-Lösung auf pH 8 eingestellt. Die Lösung wurde anschließend mit Dichlormethan extrahiert und die organische Phase einrotiert. Es wurden 13 g 2-Acetoxymethyl-5-methylpyridin erhalten.

Dieses wurde in 90%igem Methanol gelöst und nach Versetzen mit 4,7 g NaOH 6 Stunden lang am Rückfluß gekocht. Die Lösung wurde anschließend mit Salzsäure auf pH 7 eingestellt und mit Dichlormethan extrahiert. Die organische Phase wurde einrotiert und 8,5 g 2-Hydroxymethyl-5-methylpyridin erhalten.

Dieses wurde in 150 ml Dichlormethan gelöst und nach Versetzen mit 8 g aktivem $MnO_2$ 7,5 Stunden lang am Rückfluß gekocht. Die Lösung wurde heiß filtriert und das Filtrat einrotiert. Es wurden 7 g 5-Methylpyridin-2-aldehyd erhalten.

0,62 g 5-Methylpyridin-2-aldehyd wurden zusammen mit 1 g 1-Hydrazinophthalazin . Hydrochlorid und 5,1 ml 1N NaOH in Methanol gelöst und nach Versetzen mit wenig Wasser 2 Stunden lang am Rückfluß gekocht.

Die Lösung wurde anschließend in heißem Zustand mit Aktivkohle filtriert und mit wenig NaOH versetzt. Die nach Abkühlen ausgefallenen Kristalle wurden abgesaugt und aus Methanol/Wasser umkristallisiert.

Es wurden 0,75 g einer gelben Substanz mit einem Schmelzpunkt von 207-210°C gewonnen.

## Beispiel 7

Herstellung von 4-Acetylamino-pyridin-2-aldehyd-1'-phthalazinylhydrazon (PH 22-18)

4 g 2-Methyl-4-nitro-pyridin-N-oxid wurden in 90 ml Methanol gelöst und mit 1,2 g 10%iger Pd/Kohle und $H_2$ reduziert. Der Katalysator wurde abfiltriert und das Filtrat einrotiert. Der Rückstand wurde mit 200 ml Essigsäureethylester ausgekocht, heiß filtriert und das Filtrat mit 100 ml Hexan versetzt. Die nach Abkühlen der Lösung ausgefallenen Kristalle wurden abgesaugt, gewaschen und getrocknet.

Es wurden 3,1 g 2-Methyl-4-aminopyridin-N-oxid erhalten.

6,5 g des so gewonnenen 2-Methyl-4-amino-pyridin-N-oxids wurden in 80 ml Acetanhydrid gelöst und 3 Stunden lang auf 80 bis 100°C erwärmt. Das überschüssige Anhydrid wurde mit Eis zersetzt, die Lösung anschließend mit $Na_2CO_3$ auf pH 8 eingestellt und mit Dichlormethan extrahiert. Nach Einrotieren der organischen Phase wurden 5 g 2-Acetoxymethyl-4-acetylamino-pyridin erhalten.

Dieses wurde in 100 ml 90%igem Methanol gelöst und nach Versetzen mit 9,3 g $Na_2CO_3.H_2O$ 17 Stunden lang bei Raumtemperatur gerührt. Die Lösung wurde anschließend mit Essigsäure neutralisiert und mit $NaHCO_3$ auf pH 7,5 eingestellt. Nach Extraktion mit Ether fiel ein Teil des gebildeten 2-Hydroxymethyl-4-acetylaminopyridins aus, der durch Absaugen gewonnen wurde. Anschließend wurde die wässrige Phase mit Essigsäureethylester extrahiert, die organische Phase auf ein Drittel eingeengt und die restliche Substanz mit Hexan ausgefällt.

Es wurden zusammen 2 g 2-Hydroxymethyl-4-acetylaminopyridin erhalten.

2 g des so gewonnenen 2-Hydroxymethyl-4-acetylaminopyridins wurden in 400 ml Dichlormethan aufgeschlämmt, mit 6 g $MnO_2$ versetzt und 26 Stunden lang am Rückfluß gekocht. Das $MnO_2$ wurde anschließend heiß abfiltriert und die Dichlormethanlösung bis zur Trockne einrotiert. Es wurden 1,5 g 4-Acetylamino-pyridin-2-aldehyd erhalten.

1,2 g 4-Acetylaminopyridin-2aldehyd wurden zusammen mit 1,5 g 1-Hydrazinophthalazin . Hydrochlorid und 7,6 ml 1H NaOH in 50 ml Methanol/$H_2O$ (1:1) gelöst und 1 Stunde lang am Rückfluß gekocht. Nach Abkühlen der Lösung und Zugeben von Wasser fiel ein Niederschlag aus, der abgesaugt und aus Methanol/$H_2O$ umkristallisiert wurde.

Es wurden 1,2 g einer gelben Substanz mit einem Schmelzpunkt von 140-143°C gewonnen.

## Beispiel 8

Herstellung von 4-Acetylamino-2-acetylpyridin-1'-phthalazinylhydrazon (PH 22-20)

Ausgehend von 2-Ethylpyridin wurde 2-Ethyl-4-nitropyridin-N-oxid hergestellt (analog zur Synthesevorschrift für 2-Methyl-4-nitropyridin-N-oxid von E. Ochiai, J. Org. Chem. 1953 (18) 534).

4 g dieser Substanz wurden in 60 ml Methanol gelöst und mit 1,2 g 10%-iger Pd/Kohle und $H_2$ reduziert. Der Katalysator wurde abfiltriert und das Filtrat einrotiert.

Es wurden 3,3 g 4-Amino-2-ethylpyridin-N-oxid erhalten.

6,5 g dieser Substanz wurden mit 50 ml Acetanhydrid 5 Stunden lang bei 60-80°C gerührt. Nach Abkühlen wurde die Mischung auf Eis gegeben und mit $Na_2CO_3$ neutralisiert. Die Lösung wurde mit Dichlormethan extrahiert und die organische Phase einrotiert. Der Rückstand wurde in 70 ml 80%-igem Methanol gelöst, mit 9 g $Na_2CO_3.H_2O$ versetzt und 20 Stunden lang bei Raumtemperatur gerührt. Nach dem Abrotieren des Methanols wurde mit Wasser versetzt und 2 Tage lang im Extractor mit Dichlormethan extrahiert. Darauf wurde die organische Phase zur Trockne einrotiert und der Rückstand aus Essigsäureethylester/Hexan umkristallisiert.

Es wurden 2 g 2-(1-Hydroxyethyl)-4-acetylaminopyridin erhalten.

2 g dieser Verbindung wurden mit 6 g $MnO_2$ in 200 ml Dichlormethan 2 Stunden lang am Rückfluß gekocht. Nach dem Filtrieren der heißen Lösung wurde mit Wasser extrahiert und die $H_2O$-Phase zur Trockne einrotiert.

Es wurden 1,1 g 2-Acetyl-4-acetylaminopyridin erhalten.

1,1 g 2-Acetyl-4-acetylaminopyridin wurden in 20 ml Methanol gelöst und mit 0,55 g 1-Hydrazinophthalazin . HCl sowie 2,7 ml 1N NaOH und 20 ml $H_2O$ versetzt und 1,5 Stunden lang am Rückfluß gekocht. Die beim Abkühlen ausfallenden Kristalle wurden aus Methanol/$H_2O$ umkristallisiert.

Es wurden 0,35 g einer gelben Substanz mit einem Schmelzpunkt von 249-253°C erhalten.

**Beispiel 9**

Herstellung von 2-Benzoylpyridin-1'-phthalazinylhydrazon (PH 22-22)

1 g 2-Benzoylpyridin, 1,1 g 2-Hydrazinophthalazin . Hydrochlorid und 5,5 ml 1N NaOH wurden in 60 ml 70%igem Methanol 3 1/2 Stunden lang am Rückfluß gekocht. Die Lösung wurde anschließend mit Aktivkohle versetzt und heiß filtriert.

Die nach Abkühlen des Filtrats ausgefallenen Kristalle wurden abgesaugt und aus Methanol/$H_2O$ umkristallisiert.

Es wurden 0,45 g einer gelben Substanz mit einem Schmelzpunkt von 177-179°C gewonnen.

**Beispiel 10**

Herstellung von 4-Aminopyridin-2-aldehyd-1'-phthalazinyl-hydrazon (PH 22-23)

0,2 g PH 22-18 wurden in einer Lösung von 50 mg NaOH in 6 ml Methanol gelöst. Die Lösung wurde 3 Tage lang bei Raumtemperatur stehen gelassen, erneut mit 50 mg NaOH/6 ml Methanol versetzt und 2 Tage lang bei Raumtemperatur stehen gelassen. Nach Zusatz von wenig Aktivkohle wurde filtriert und die Substanz durch Zugabe von Wasser ausgefällt, abgesaugt und aus Methanol/Wasser umkristallisiert.

Es wurden 55 mg einer gelben Substanz mit einem Schmelzpunkt von 252-255°C gewonnen.

**Beispiel 11**

Herstellung von 2-Acetyl-4-aminopyridin-1'-phthalazinylhydrazon (PH 22-24)

0,25 g PH 22-20 wurden umgesetzt und isoliert wie in Beispiel 10 beschrieben.

Es wurden 100 mg einer gelben Substanz mit einem Schmelzpunkt von 232-234°C erhalten.

**Beispiel 12**

Herstellung von Ethyl-2-pyridylketon-1'-phthalazinylhydrazon (PH 22-25)

20 ml 2-n-Propylpyridin wurden in 60 ml Eisessig gelöst, mit 40 ml $H_2O_2$ (30%-ig) versetzt und 16 Stunden lang bei Raumtemperatur sowie 3 Stunden lang bei 65°C gerührt. Darauf wurde die Essigsäure teilweise abrotiert, der Rückstand mit NaOH (20%-ig) auf pH 8 eingestellt, mit Dichlormethan extrahiert und die organische Phase zur Trockne einrotiert.

Es wurden 20 g rohes 2-Propylpyridin-N-oxid erhalten.

19 g 2-Propylpyridin-N-oxid wurden in 40 ml Eisessig/40 ml Acetanhydrid gelöst und 6 Stunden lang bei 85°C sowie 12 Stunden lang bei Raumtemperatur gerührt. Überschüssiges Anhydrid wurde mit $H_2O$ zersetzt, die Lösung mit $Na_2CO_3$ neutralisiert und mit Diethylether extrahiert. Nach dem Ein-rotieren der Etherphase verblieben 12 g 2-(1-Acetoxypropyl)-pyridin. Dieses Produkt wurde in 120 ml Methanol (70%-ig) gelöst, mit 4 g NaOH versetzt und 6 Stunden am Rückfluß gekocht. Nach Abrotieren des Methanols wurde mit Wasser versetzt, mit Diethylether extrahiert und die organische Phase einrotiert. Es verblieben 5,4 g 2-(1-Hydroxypropyl)-pyridin. 0,5 g dieser Substanz wurden mit 1 g $MnO_2$ und 30 ml Dichlormethan 2 Stunden am Rückfluß gekocht. Nach Abfiltrieren des $MnO_2$ wurde zur Trockne einrotiert.

Es wurden 0,3 g Ethyl-2-pyridylketon erhalten.

0,8 g dieser Substanz wurden in 60 ml Methanol/$H_2O$ (1:1) mit 1,16 g 1-Hydrazinophthalazin Hydrochlorid und 6 ml 1N NaOH 2 Stunden am Rückfluß gekocht. Die Lösung wurde mit Aktivkohle versetzt, heiß filtriert und abgekühlt. In der Kälte kristallisierte das Produkt aus.

Es wurden 0,41 g einer gelben Substanz mit einem Schmelzpunkt von 143-145°C erhalten.

**Beispiel 13**

Herstellung von Pyridin-2-aldehyd-6'-chlor-4'-pyrimidylhydrazon (PH 22-26)

12 g 4,6-Dichlorpyrimidin wurden in 100 ml Methanol gelöst, tropfenweise mit 12 ml Hydrazinhydrat (80%-ig) versetzt und 1 Stunde bei Raumtemperatur gerührt. Nach Absaugen des Niederschlags und Waschen mit $H_2O$ wurden 10 g 4-Hydrazino-6-chlorpyrimidin erhalten.

10 g dieser Substanz wurden in 130 ml Methanol und 50 ml Dichlormethan suspendiert, mit 8,2 g Pyridin-2-aldehyd versetzt und 2 Stunden lang bei Raumtemperatur stehen gelassen. Die sich ausscheidenden Kristalle wurden abgesaugt und mit Methanol gewaschen.

Es wurden 15,1 g einer hellgelben Substanz mit einem Schmelzpunkt von 264-266°C erhalten.

**Beispiel 14**

Herstellung von Pyridin-2-aldehyd-6'-(N-methyl-N-2''-hydroxypropyl)amino-3'-pyridazinylhydrazon (PH 22-28)

0,45 g 3-Hydrazino-6-(N-methyl-N-2'-hydroxypropyl)aminopyridazin (L. Dorigotti et al., Arzneim.-Forsch. **34**, 876 (1984)) wurden in 10 ml Methanol gelöst und mit 0,28 g Pyridin-2-aldehyd versetzt. Nach 2 Stunden langem Rühren bei Raumtemperatur wurde zur Trockne einrotiert und aus Methanol/Wasser umkristallisiert.

Es wurden 0,36 g einer gelben Substanz mit einem Schmelzpunkt von 208-210°C erhalten.

**Beispiel 15**

Herstellung von 2-Acetylpyridin-6'-chlor-4'-pyrimidylhydrazon(PH 22-32)

1 g 6-chlor-4-hydrazinopyrimidin gemäß Beispiel 13 wurde in 100 ml Methanol gelöst, mit 0,83 g 2-Acetylpyridin versetzt und 18 Stunden lang bei Raumtemperatur gerührt. Die Lösung wurde auf ca 1/3 des Volumens einrotiert und das Produkt mit $H_2O$ ausgefällt, abgesaugt und aus Methanol/Wasser umkristallisiert.

Es wurden 1,6 g einer hellgelben Substanz mit einem Schmelzpunkt von 145-148°C erhalten.

**Beispiel 16**

Herstellung von 5-Ethylpyridin-2-aldehyd-6'-methoxy-4'-pyrimidylhydrazon (PH 22-33)

3 g 4,6-Dichlorpyrimidin wurden in 30 ml getrocknetem Methanol gelöst und bei -10°C tropfenweise mit einer Lösung von 0,46 g Natrium in 20 ml getrocknetem Methanol versetzt und 5 Stunden lang bei -10°C sowie 12 Stunden lang bei Raumtemperatur gerührt. Die Lösung wurde einrotiert, der Rückstand mit 50 ml Dichlormethan aufgenommen und etwas Aktivkohle zugesetzt.

Nach dem Filtrieren wurde zur Trockne einrotiert. Der zunächst flüssige Rückstand wurde beim Stehen kristallin fest.

Es wurden 2,5 g 4-Chlor-6-methoxy-pyrimidin erhalten.

2,5 g dieser Substanz wurden in 15 ml Methanol gelöst, mit 6 ml Hydrazinhydrat (80%-ig) versetzt und 1 Tag lang am Rückfluß gekocht. Nach Einrotieren wurde mit wenig Wasser aufgenommen, mit 1N NaOH auf pH 10 eingestellt und mit Dichlormethan extrahiert. Die organische Phase wurde zur Trockne eingeengt.

Es wurden 0,92 g 4-Hydrazino-6-methoxypyrimidin erhalten.

0,9 g dieser Substanz wurden in 70 ml Ethanol gelöst und mit 0,96 g 5-Ethylpyridin-2-aldehyd gemäß Beispiel 18 und einigen Tropfen Eisessig versetzt und 0,5 Stunden lang am Rückfluß gekocht. Nach Abkühlen wurde das Volumen auf die Hälfte einrotiert und das Produkt mit $H_2O$ ausgefällt und aus Ethanol/Wasser umkristallisiert.

Es wurden 0,5 g einer gelblich-braunen Substanz mit einem Schmelzpunkt von 190-192°C erhalten.

## Beispiel 17

Herstellung von 2-Acetylpyridin-4'-chinazolylhydrazon (PH 22-34)

9,2 g 4-Hydrazinochinazolin wurden in 500 ml Methanol gelöst, mit 6,9 g 2-Acetylpyridin versetzt und 2 Stunden lang am Rückfluß gekocht. Nach Abkühlen wurde das Produkt mit Wasser ausgefällt und aus Methanol/Wasser umkristallisiert.

Es wurden 14 g einer gelben Substanz mit einem Schmelzpunkt von 198-199°C erhalten.

## Beispiel 18

Herstellung von 2-Acetylpyridin-6'-chlor-4'-pyrimidyl-($N_1$-methyl)-hydrazon (PH 22-35)

1,2 g 6-Chlor-4-(1-methyl-hydrazino)-pyrimidin (s. PH 22-36) wurden in 30 ml Methanol gelöst und mit 0,92 g 2-Acetylpyridin in 20 ml Methanol versetzt. Nach kräftigem Rühren wurde 2 Tage lang im Dunkeln bei Raumtemperatur stehen gelassen und 2 Tage lang am Rückfluß gekocht. Anschließend wurde die Lösung auf ca 50% des Volumens eingeengt und mit Wasser versetzt. Die ausgefallenen Kristalle wurden aus Methanol/$H_2O$ umkristallisiert.

Es wurden 0,43 g einer hellgelben Substanz mit einem Schmelzpunkt von 111-114°C erhalten.

## Beispiel 19

Herstellung von Pyridin-2-aldehyd-6'-chlor-4'-pyrimidyl-($N_1$-methyl)-hydrazon (PH 22-36)

2 g 4,6-Dichlorpyrimidin wurden in 30 ml Methanol gelöst, mit 1,37 g Triethylamin und 0,63 g Methylhydrazin versetzt und 2 Tage lang bei Raumtemperatur gerührt. Danach wurden 0,1 ml Methylhydrazin und 0,2 ml Triethylamin zugesetzt und 4 Tage lang bei Raumtemperatur gerührt. Nach dem Abrotieren des Lösungsmittels wurde ein Rückstand von 2,4 g rohem 6-Chlor-4-(1-methyl-hydrazino)-pyrimidin erhalten, welcher sofort weiter verarbeitet wurde.

1,2 g dieser Substanz wurden in 30 ml Methanol gelöst und mit 0,82 g Pyridin-2-aldehyd in 20 ml Methanol versetzt. Die Lösung wurde 3 Tage lang im Dunkeln bei Raumtemperatur stehen gelassen und dann auf 1/3 des Volumens eingeengt. Die ausgefallenen Kristalle wurden aus Methanol/$H_2O$ umkristallisiert.

Es wurden 0,35 g einer hellgelben Substanz mit einem Schmelzpunkt von 182-185°C erhalten.

Die in den nachfolgenden Beispielen verwendeten Mikroorganismenstämme sind unter den angegebenen Nummern in der Stammsammlung des Forschungsinstituts Borstel, Parkallee 1 - 42, 2061 Borstel hinterlegt.

## Beispiel 20

Die akute Toxizität nach einmaliger Verabreichung von PH 22 Pyridin-2-aldehyd-thiosemicarbazon (Py-2-TSC) sowie Conteben (Thiacetazon, 4-Acetylaminobenzaldehyd-thiosemicarbazon) wurde nach H.J. Hapke, Toxikologie für Veterinärmediziner, F. Enke Verlag, Stuttgart 1975, ermittelt. Als Versuchstiere wurden WISTAR-Ratten und NMRI-weiße Mäuse verwendet. Die beobachteten Toxizitäten sind mit einigen in der Literatur gefundenen Daten verglichen und vorne in Tabelle 1 wiedergegeben. Außerdem wurden bei einer 12-monatigen Verfütterung von PH 22-26 (0,1% im Futter) während eines Therapieversuchs bei Lepra-infizierten Mäusen (s.Tab. 17) keine toxischen Effekte beobachet (Gewichtskonstanz).

**Beispiel 21**

Nachweis der Hemmwirkung der erfindungsgemäßen Substanzen gegenüber Mycobakterien.

Die minimale Hemmkonzentration der in Tabelle 2 angegebenen handelsüblichen und erfindungsgemäßen Substanzen wurde nach bekannten Standardverfahren ("Methoden zur Empfindlichkeitsprüfung von bakteriellen Krankheitserregern", DIN 58940, Teil 5) bestimmt.

Die Bestimmung wurde an den folgenden Bakterienkulturen vorgenommen:

1. Mycobacterium lufu (F. Portaels, Annls. Soc. belg. Méd. trop. 60, 381 (1980)), FIB 1-85 JS
2. Mycobacterium tuberculosis H 37 RV, FIB 2-85 JS
3. Mycobacterium marinum SN 1254, FIB 3-85 JS
4. Mycobacterium smegmatis ATCC 607 FIB 5-85 JS
5. Mycobacterium avium SN 304 FIB 6-85 JS
6. Mycobacterium avium Patientenstamm (Birk) FIB 1-88 JS

Zur Anzucht der Bakterienkulturen wurden Nährböden nach Gottsacker und Löwenstein Jensen sowie Nährmedien nach Lockemann oder Dubos-Davis verwendet (vgl. "Nachweisverfahren für Mycobakterien aus Untersuchungsmaterial, III, Nährbodenrezepte zur Kultur von Tuberkulosebakterien", 1978, Herausgeber: Deutsches Zentralkomitee zur Bekämpfung der Tuberculose, Poppenhusenstraße 14c, 2000 Hamburg 60).

Jeweils 5 x $10^{-3}$ bis 5 x $10^{-5}$ mg, bezogen auf Feuchtgewicht, der Mycobakterienstämme wurden in 2 ml Kulturflüssigkeit (Lockemann + 0,5 Gew./Vol.% Rinderserumalbumin-Fraktion 5) bei 31°C mit abgestuften Konzentrationen des jeweiligen Hemmstoffs (Verdünnungsreihe) inkubiert. Die Ablesung der Kulturröhrchen erfolgte je nach Bakterienstamm nach 8 bis 15 Tagen.

Die MHK ist diejenige Konzentration des Hemmstoffs in µMol/l, bei der keine Vermehrung der eingesäten Zellen festgestellt wird.

Die Ergebnisse sind in Tabelle 2 wiedergegeben. Sie zeigen, daß die erfindungsgemäßen Verbindungen eine gegenüber den Vergleichspräparaten bis um mehr als das 200-Fache gesteigerte antimycobakterielle Wirksamkeit aufweisen.

Tabelle 2

| Minimale Hemmkonzentration [µM/l] | | | | | | |
|---|---|---|---|---|---|---|
| Substanz | M. lufu L 209 | M. tub. H37RV | M. marin. SN1254 | M. smegm. ATCC 607 | M. avium SN 304 | M. avium Patientenst. |
| Bactrim | | | | | 32 | >64* |
| Brodimoprim | 80 | 94 | 18 | | - | >100* |
| Streptomycin | - | | - | | 55 | - |
| Kanamycin | | | | | 33 | 17 |
| Rifampicin | | | | | 39 | 5 |
| Conteben | 339 | 4 | | | | 35 |
| Py-2-TSC** | 40 | | | | | |
| PH 22-1 | 12,1 | 49 | | 49 | | |
| PH 22-3 | 6,6 | 31 | 22 | 44 | | 53 |
| PH 22-5 | 14,5 | 16,6 | 10,4 | 67 | | |
| PH 22-6 | 5,6 | 8,5 | 7 | 40 | | 7 |
| PH 22-8 | 31 | 18 | 22 | 44 | | |
| PH 22-16 | 4,8 | 1,4 | 76 | 5,7 | | |
| PH 22-18 | 22,9 | 46 | 92 | 65 | | |
| PH 22-20 | 12,5 | 10,9 | 88 | 15,6 | | |
| PH 22-22 | 2,3 | 18,5 | 10,8 | 12,3 | | 86 |
| PH 22-23 | 26,5 | 13,3 | 38 | 13,3 | | |
| PH 22-24 | 2,2 | 4,5 | 18 | 4,5 | | |
| PH 22-25 | 2,7 | 36 | 101 | 10,8 | | |
| PH 22-26 | 1,3 | 6,2 | 12,9 | 25,7 | | |
| PH 22-28 | 35 | 140 | 70 | | | |
| PH 22-32 | 1,3 | 6,1 | 28,3 | | | |
| PH 22-33 | 1,5 | 2,4 | 4,9 | | | |
| PH 22-34 | <1,0 | 12 | 64 | | | |
| PH 22-36 | 5,3 | 136 | 119 | | | |

* µg/ml
** Py-2-TSC = Pyridin-2-aldehyd-thio-semicarbazon

**Beispiel 22**

Bestimmung der minimalen Hemmkonzentration (MHK) von Kombinationen der erfindungsgemäßen Verbindungen mit Hemmstoffen der Folatsynthase und Dihydrofolsäurereduktase, DNA-Synthese und RNA-Synthese.

Die MHK der Kombinationen wurde für Mycobakterium lufu durch Schachbrett-Titrationen (vgl. Behrenbaum M.C., J. Infect. Dis. 137, 122 bis 130 (1978) und J.K.Seydel et al., Chemotherapy, 29,249,(1983)) bestimmt.

Die Bakterien wurden unter den Bedingungen gemäß Beispiel 21 inkubiert.

Es wurde eine 2-dimensionale Verdünnungsreihe hergestellt, wobei die jeweiligen Komponenten in den Mengen vorlagen, wie in den Tabellen 3 bis 6 angegeben.

Die Ergebnisse sind in den Tabellen 3 und 6 wiedergegeben.

In den Tabellen bedeuten

DDS = 4,4'-Diaminodiphenylsulfon

K 107 = 2,4-Diamino-5-(4-[2-(4-aminophenyl-4-sulfonylphenylamino)ethoxy]-3,5-dimethoxybenzyl)pyrimidin

K 130 = 2,4-Diamino-5-(4-[3-(4'-aminophenyl-4-sulfonylphenylamino)-propoxy]-3,5-dimethoxybenzyl)-pyrimidin

Die Verbindungen K 107 und K 130 sind in der DE-OS 36 03 577 offenbart.

+ + + = Wachstum wie Kontrolle
+ + = leichte Wachstumshemmung
+ = starke Wachstumshemmung
(+) = nahezu vollständige Wachstumshemmung

- = vollständige Wachstumshemmung.

**Beispiel 23**

Nachweis der Hemmwirkung der erfindungsgemäßen Substanz allein und in Kombination gegenüber M. leprae (in vitro)

Die Hemmwirkung wurde als Reaktion der Thymidin-Aufnahme und des ATP-Gehalts in M. Leprae-Suspensionen bestimmt wie von A.M. Dhople, Med. Sci. Res. **15**, 599 (1987) beschrieben. Die Ergebnisse sind in Tabelle 7 wiedergegeben.

**Beispiel 24**

Nachweis der therapeutischen Wirkung der erfindungsgemäßen Substanzen allein und in Kombination in Lepra-infizierten Mäusen.

Die therapeutische Wirkung wurde als Reduktion der Zahl der säure-festen Bakterien in der Mäusepfote ermittelt nach C.C. Shepard, J. Experim. Med. 112, 445 (1960).

Die Ergebnisse für die Monotherapie sind in Tabelle 8 und für die Kombination in Tabelle 9 wiedergegeben. Auch unter in vivo-Bedingungen ist der synergistische Effekt der Kombinationen nachweisbar.

**Tabelle 3**

PH 22-26 [µg/ml] ←

| DDS [µg/ml] ↑ | 0,8 | 0,7 | 0,6 | 0,5 | 0,4 | 0,3 | 0,2 | 0,1 | 0 |
|---|---|---|---|---|---|---|---|---|---|
| 0,015 | | | | | | | | | - |
| 0,0135 | | | | | | | | | - |
| 0,012 | | | | | | | | - | + |
| 0,0105 | | | | | | | | - | ++ |
| 0,009 | | | | | | | | - | ++ |
| 0,0075 | | | | | | | | - | +++ |
| 0,006 | | | | | | | | - | ++ |
| 0,0045 | | | | | | | - | (+) | +++ |
| 0,003 | | | | | | | - | + | +++ |
| 0,0015 | | | - | - | - | - | - | ++ | +++ |
| 0 | - | - | (+) | + | ++ | ++ | +++ | +++ | |

M. lufu L209

**Tabelle 4**

PH 22-26 [µg/ml] ←

| K 130 [µg/ml] ↑ | 0,9 | 0,8 | 0,7 | 0,6 | 0,5 | 0,4 | 0,3 | 0,2 | 0,1 | 0 |
|---|---|---|---|---|---|---|---|---|---|---|
| 1 | | | | | | | | | | - |
| 0,9 | | | | | | | | | | - |
| 0,8 | | | | | | | | | | - |
| 0,7 | | | | | | | | | - | (+) |
| 0,6 | | | | | | | | | - | (+) |
| 0,5 | | | | | | | | | - | + |
| 0,4 | | | | | | | | - | (+) | + |
| 0,3 | | | | | | | | - | (+) | ++ |
| 0,2 | | | | | | | | - | (+) | +++ |
| 0,1 | | | - | - | - | - | - | (+) | ++ | +++ |
| 0 | - | - | + | + | ++ | +++ | +++ | +++ | +++ | |

M. lufu L 209

**Tabelle 5**

5-F-Uracil [µg/ml] ←

| PH 22-26 [µg/ml] ↑ | 16 | 14,4 | 12,8 | 11,2 | 9,6 | 8 | 6,4 | 4,8 | 3,2 | 1,6 | 0 |
|---|---|---|---|---|---|---|---|---|---|---|---|
| 0,4 | | | | | | | | | | | - |
| 0,36 | | | | | | | | | | | - |
| 0,32 | | | | | | | | | | - | (+) |
| 0,28 | | | | | | | | | | - | (+) |
| 0,24 | | | | | | | | | | - | + |
| 0,2 | | | | | | | | | | - | ++ |
| 0,16 | | | | | | | | | | - | ++ |
| 0,12 | | | | | | | | | - | - | +++ |
| 0,08 | | | | - | - | - | - | - | (+) | + | +++ |
| 0,04 | | - | - | - | (+) | (+) | + | + | + | ++ | +++ |
| 0 | - | - | (+) | (+) | + | + | ++ | +++ | +++ | +++ | |

M. lufu L 209

**Tabelle 6**

PH 22-26 [µg/ml] ←

| Rifampicin [µg/ml] ↑ | 0,8 | 0,72 | 0,64 | 0,56 | 0,48 | 0,4 | 0,32 | 0,24 | 0,16 | 0,08 | 0 |
|---|---|---|---|---|---|---|---|---|---|---|---|
| 0,08 | | | | | | | | | | | - |
| 0,072 | | | | | | | | | | - | (+) |
| 0,064 | | | | | | | | | | - | (+) |
| 0,056 | | | | | | | | | - | (+) | + |
| 0,048 | | | | | | | | - | (+) | (+) | + |
| 0,04 | | | | | | | | - | (+) | + | ++ |
| 0,032 | | | | | | | - | (+) | + | + | ++ |
| 0,024 | | | | | | | - | (+) | + | ++ | +++ |
| 0,016 | | | | | - | - | (+) | + | + | ++ | +++ |
| 0,008 | - | - | - | - | (+) | + | ++ | ++ | ++ | +++ | +++ |
| 0 | (+) | (+) | + | ++ | +++ | +++ | +++ | +++ | +++ | +++ | |

M. lufu L 209

Tabelle 7

| Effekt von Wirkstoff-Kombinationen auf die in vitro-Vermehrung von M. leprae Wirkstoffkombination (mg/ml) | | | | |
|---|---|---|---|---|
| DDS | K-130 | Rifampicin | PH 22-26 | % Reduktion Thymidin-Einbau |
| 5 | | | | 0 |
| 10 | | | | 5 |
| | 10 | | | 0 |
| | 20 | | | 65 |
| | | 50 | | 2 |
| | | 100 | | 0 |
| | | | 50 | 30 |
| | | | 100 | 48 |
| 10 | | | 50 | 37 |
| 10 | | | 100 | 57 |
| | 20 | | 50 | 61 |
| | 20 | | 100 | 73 |
| | | 50 | 50 | 34 |
| | | 50 | 100 | 54 |
| | | 100 | 50 | 47 |
| | | 100 | 100 | 65 |

Tabelle 8

| Hemm-Effekt von PH 22-26 auf die Vermehrung von M. leprae in Mäusepfoten M. leprae/Pfote (x $10^6$) | | | |
|---|---|---|---|
| Behandlungsdauer | Kontrolle | behandelte Mäuse (0,1% im Futter) | % Hemmung |
| 4 Monate | 0,468 | 0,283 | 40 |
| 6,5 Monate | 1,720 | 0,540 | 69 |
| 8,5 Monate | 2,978 | 0,684 | 77 |
| 12 Monate | 3,682 | 0,945 | 74 |

Tabelle 9

| Hemm-Effekt von Wirkstoff-Kombinationen auf die Vermehrung von M. leprae in Mäusepfoten nach 2-monatiger Therapie (Applikation im Futter) | | | | | |
|---|---|---|---|---|---|
| DDS | Rifampicin | K-130 | Brodimoprim | PH 22-26 | % Hemmung |
| 0,0001% | | | | | 14 |
| | 0,001% | | | | 19 |
| | | 0,01% | | | 56 |
| | | | 0,01% | | 3 |
| | | | | 0,05% | 0 |
| 0,0001% | | | | 0,05% | 8 |
| | 0,001% | | | 0,05% | 38 |
| | | | 0,01% | 0,05% | 5 |
| 0,0001% | | | 0,01% | 0,05% | 10 |
| 0,0001% | | 0,01% | | 0,05% | 81 |
| 0,0001% | 0,001% | | | 0,05% | 86 |

**Beispiel 25**

Bestimmung der halbmaximalen Hemmkonzentration ($I_{50}$) von typischen Vertretern der erfindungsgemäßen Verbindungen für das Wachstum der parasitären Malariastämme
Indochina W-2
Sierra Leone D-6

Die Bestimmung wurde nach Standardverfahren vorgenommen (vgl. WHO-Bestimmungsverfahren).
Die Ergebnisse sind in Tabelle 10 wiedergegeben.

Tabelle 10

| | Indochina W-2 | | | Sierra Leone D-6 | |
|---|---|---|---|---|---|
| | $I_{50}$ | $I_{90}$ | [µM/l] | $I_{50}$ | $I_{90}$ |
| PH 22-25 | 0.4 | 0.7 | | 0.3 | 0.45 |
| PH 22-26 | 3.5 | 6.2 | | 2 | 3 |

**Beispiel 26**

In-vitro-Bestimmung der cytostatischen Wirksamkeit von typischen Vertretern der erfindungsgemäßen Verbindungen

Die halbmaximale Hemmung der Zellzahlzunahme sowie des Thymidineinbaus in die DNA wurde an der hormonunabhängigen humanen Mammatumorzellinie MDA-MB231 (vgl. H. D. Soule u.a. J.Nat.Cancer Inst. 51, 1409 (1973)) nach der von H. Seidenberger, G. Kranzfelder, S. Wappes, H. Schönenberger, W.Beck und M. Girth, Arch. Pharm. 314, 955 (1981) sowie von M. Lippman , M.E. Monaco und G. Bolan, Cancer Res. 37 ,1901 (1977) beschriebenen Methode bestimmt.
Die Ergebnisse sind nachfolgend in der Tabelle 11 wiedergegeben.

Tabelle 11

| | 50% Hemmung der Zellzahlzunahme $I_{50}$µM/l | 50% Hemmung des Thymidin-Einbaus |
|---|---|---|
| PH 22-25 | 0.194 | 0.104 |

**Patentansprüche**

1. Arzneimittel mit einem Gehalt an substituierten 2-Acylpyridin-α-(N)-hetarylhydrazonen der allgemeinen Formel I

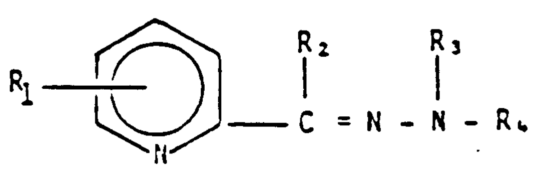

I

in der $R_1$ Wasserstoff, Halogen, eine Alkylgruppe mit 1 bis 4 C-Atomen, eine Benzyloxy-, Benzo-, Amino-, oder Acetaminogruppe,
$R_2$ Wasserstoff, eine Alkylgruppe mit 1 bis 8 C-Atomen, eine Amino oder eine Phenylgruppe,

$R_3$ Wasserstoff oder eine Methylgruppe und
$R_4$ ein Rest der allgemeinen Formeln II oder III

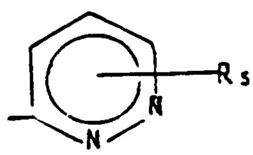

II

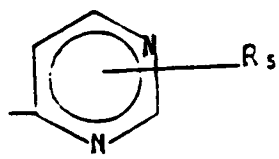

III

ist, in denen
$R_5$ Wasserstoff, Halogen, eine Alkyl- oder Alkoxygruppe mit 1 bis 4 C-Atomen, eine Benzogruppe oder eine Dialkylamino- oder substituierte Dialkylaminogruppe mit 1 bis 3 C-Atomen in den Alkylresten ist, als therapeutischem Wirkstoff.

2. Arzneimittel nach Anspruch 1, **dadurch gekennzeichnet**, daß es als therapeutischen Wirkstoff eine Verbindung enthält, in der $R_1$ und $R_2$ unabhängig voneinander Aminogruppen, Wasserstoff oder Alkylgruppen mit 1 bis 4 C-Atomen sind.

3. Arzneimittel nach den Ansprüchen 1 oder 2, **dadurch gekennzeichnet**, daß es als therapeutischen Wirkstoff eine Verbindung enthält, in der $R_1$ eine Benzyloxy-, Benzo- oder Acetaminogruppe ist.

4. Arzneimittel nach den Ansprüchen 1 bis 3, **dadurch gekennzeichnet**, daß es als therapeutischen Wirkstoff eine Verbindung enthält, in der $R_2$ eine Phenylgruppe ist.

5. Arzneimittel nach den Ansprüchen 1 bis 4, **dadurch gekennzeichnet**, daß es als therapeutischen Wirkstoff eine Verbindung enthält, in der $R_5$ eine (N)-Methyl-(N)-2-hydroxypropylamino-,Methyl-, Chlor-, Methoxy- oder Benzogruppe ist.

6. Arzneimittel nach Anspruch 1, **dadurch gekennzeichnet**, daß es als therapeutischen Wirkstoff eine der nachfolgenden Verbindungen enthält, in denen
$R_1$, $R_2$ und $R_3$ Wasserstoff und $R_4$ ein 3'-Pyridazinylrest ist, in dem $R_5$ eine 4,5'-Benzogruppe ist (PH 22-1),

oder $R_1$ und $R_3$ Wasserstoff sind, $R_2$ eine Methylgruppe und $R_4$ ein 3'-Pyridazinylrest sind, in dem $R_5$ eine 4',5'-Benzogruppe ist (PH 22-3),

oder $R_1$ eine 5-Ethylgruppe, $R_2$ und $R_3$ Wasserstoff und $R_4$ ein 3'-Pyridazinylrest sind, in dem $R_5$ eine 4',5'-Benzogruppe ist (PH 22-5),

oder $R_1$ eine 5-Benzyloxygruppe, $R_2$ und $R_3$ Wasserstoff und $R_4$ eine 3'-Pyridazinylgruppe sind, in der $R_5$ eine 4',5'-Benzogruppe ist (PH 22-6),

oder $R_3$ eine 4-Methylgruppe, $R_2$ und $R_3$ Wasserstoff und $R_4$ ein 3'-Pyridazinylrest sind, in dem $R_5$ die 4',5'-Benzogruppe ist (PH 22-8),

oder $R_1$ eine 5-Methylgruppe, $R_2$ und $R_3$ Wasserstoff und $R_4$ ein 3'-Pyridazinylrest sind, in dem $R_5$ eine 4',5'-Benzogruppe ist (PH 22-16),

oder $R_1$ eine 4-Acetaminogruppe, $R_2$ und $R_3$ Wasserstoff und $R_4$ ein 3'-Pyridazinylrest sind, in dem $R_5$ eine 4',5'-Benzogruppe ist (PH 22-18),

oder $R_1$ eine 4-Acetaminogruppe, $R_2$ eine Methylgruppe, $R_3$ Wasserstoff und $R_4$ ein 3'-Pyridazinylrest sind, in dem $R_5$ die 4',5'-Benzogruppe ist (PH 22-20),

oder $R_1$ und $R_3$ Wasserstoff, $R_2$ ein Phenylrest und $R_4$ ein 3'-Pyridazinylrest sind, in dem $R_5$ eine 4',5'-Benzogruppe ist (PH 22-22),

oder $R_1$ eine 4-Aminogruppe, $R_2$ und $R_3$ Wasserstoff und $R_4$ ein 3'-Pyridazinylrest sind, in dem $R_5$ eine 4',5'-Benzogruppe ist (PH 22-23),

oder $R_1$ eine 4-Aminogruppe, $R_2$ eine Methylgruppe, $R_3$ Wasserstoff und $R_4$ ein 3'-Pyridazinylrest sind, in dem $R_5$ eine 4',5'-Benzogruppe ist (PH 22-24),

oder $R_1$ und $R_3$ Wasserstoff, $R_2$ eine Ethylgruppe und $R_4$ ein 3'-Pyridazinylrest sind, in dem $R_5$ eine 4',5'-Benzogruppe ist (PH 22-25),

oder $R_1$, $R_2$, $R_3$ Wasserstoff und $R_4$ ein 6'-Pyrimidinylrest sind, in dem $R_5$ 4'-Chlor ist (PH 22-26),

oder $R_1$, $R_2$, $R_3$ Wasserstoff und $R_4$ ein 3'-Pyridazinylrest sind, in dem $R_5$ eine 6'-(N)-Methyl-(N)-2-hydroxypropylaminogruppe ist (PH 22-28),

oder $R_1$ und $R_3$ Wasserstoff, $R_2$ eine Methylgruppe und $R_4$ ein 6'-Pyrimidinylrest sind, in dem $R_5$ 4'-Chlor ist (PH 22-32),

oder $R_1$ eine 5-Ethylgruppe, $R_2$ und $R_3$ Wasserstoff und $R_4$ ein 6'-Pyrimidinylrest sind, in dem $R_5$ eine 4'-Methoxygruppe ist (PH 22-33),

oder $R_1$ und $R_3$ Wasserstoff, $R_2$ eine Methylgruppe und $R_4$ ein 6'-Pyrimidinylrest sind, in dem $R_5$ eine 4',5'-Benzogruppe ist (PH 22-34),

oder $R_1$ Wasserstoff ist, $R_2$ und $R_3$ eine Methylgruppe und $R_4$ ein 6'-Pyrimidinylrest sind, in dem $R_5$ 4'-Chlor ist (PH 22-35),

oder $R_1$ und $R_2$ Wasserstoff, $R_3$ eine Methylgruppe und $R_4$ ein 6'-Pyrimidinylrest sind, in dem $R_5$ 4'-Chlor ist (PH 22-36).

**7.** Arzneimittel nach den Ansprüchen 1 bis 6 zur Behandung von mikrobiellen, insbesondere mycobakteriellen Erkrankungen oder von Malaria oder malignen Tumoren.

**8.** Arzneimittel nach den Ansprüchen 1 bis 7, **dadurch gekennzeichnet**, daß es zusätzlich einen oder mehrere Hemmstoffe der Folatsynthase und/oder der Dihydrofolsäurereduktase und/oder der DNA-Synthese und/oder RNA-Synthese enthält.

**9.** Arzneimittel nach Anspruch 8, **dadurch gekennzeichnet**, daß es als Hemmstoff der Folatsynthase ein Sulfon oder ein Sulfonamid enthält.

**10.** Arzneimittel nach Anspruch 8, **dadurch gekennzeichnet**, daß es als Hemmstoff der Dihydrofolsäurereduktase Methotrexat, Brodimoprim, 2,4-Diamino-5-{4-[2-(4'Aminophenyl-4-sulfonylphenylamino)-ethoxy]3,5-dimethoxybenzyl}pyrimidin oder 2,4-Diamino-5-{4-[2-(4'-Aminophenyl-4-sulfonylphenylamino) propoxy]3,5-dimethoxybenzyl}pyrimidin enthält.

**11.** Arzneimittel nach Anspruch 8, **dadurch gekennzeichnet**, daß es als Hemmstoff der DNA-Synthese 5-F-Uracil, Guanazol oder Desferal enthält.

**12.** Arzneimittel nach Anspruch 8, **dadurch gekennzeichnet**, daß es als Hemmstoff der RNA-Syntheise Rifampicin oder Derivate desselben enthält.

## Claims

**1.** Drug containing substituted 2-acylpyridine-α-(N)-hetarylhydrazones having the general formula I

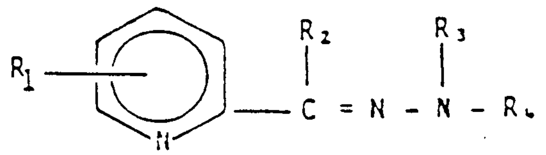

I

in which $R_1$ is hydrogen, halogen, an alkyl group with 1 to 4 C atoms, a benzyloxy, benzo, amino or acetamino group,
$R_2$ is hydrogen, an alkyl group with 1 to 8 C atoms, an amino or a phenyl group,
$R_3$ is hydrogen or a methyl group and
$R_4$ is a radical having the general formulae II or III

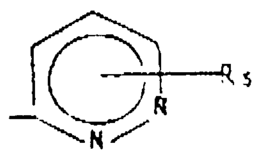

II

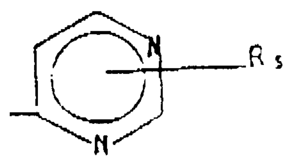

III

in which
$R_5$ is hydrogen, halogen, an alkyl or alkoxy group with 1 to 4 C atoms, a benzo group or a dialkylamino or substituted dialkylamino group with 1 to 3 C atoms in the alkyl radicals, as therapeutic active ingredient.

**2.** Drug according to claim 1, characterised in that it contains as therapeutic active ingredient a compound in which $R_1$ and $R_2$ independently of each other are amino groups, hydrogen or alkyl groups with 1 to 4 C atoms.

**3.** Drug according to claims 1 or 2, characterized in that it contains as therapeutic active ingredient a compound in which $R_1$ is a benzyloxy, benzo or acetamino group.

**4.** Drug according to claims 1 to 3, characterised in that it contains as therapeutic active ingredient a compound in which $R_2$ is a phenyl group.

**5.** Drug according to claims 1 to 4, characterised in that it contains as therapeutic active ingredient a compound in which $R_5$ is an (N)-methyl-(N)-2-hydroxypropylamino, methyl, chloro, methoxy or benzo group.

**6.** Drug according to claim 1, characterized in that it contains as therapeutic active ingredient one of the following compounds, in which
$R_1$, $R_2$ and $R_3$ is hydrogen and $R_4$ is a 3'-pyridazinyl radical, in which $R_5$ is a 4',5'-benzo group ((PH 22-1),

or $R_1$ and $R_3$ are hydrogen, $R_2$ is a methyl group and $R_4$ is a 3'-pyridazinyl radical, in which $R_5$ is a 4',5'-benzo group (PH 22-3),

or $R_1$ is a 5-ethyl group, $R_2$ and $R_3$ are hydrogen and $R_4$ is a 3'-pyridazinyl radical, in which $R_5$ is a 4',5'-benzo group (PH 22-5),

or $R_1$ is a 5-benzyloxy group, $R_7$ and $R_3$ are hydrogen and $R_4$ is a 3'-pyridazinyl group, in which $R_5$ is a 4',5'-benzo group (PH 22-6),

or $R_3$ is a 4-methyl group, $R_2$ and $R_3$ are hydrogen and $R_4$ is a 3'-pyridazinyl radical, in which $R_5$ is the 4',5'-benzo group (PH 22-8),

or $R_1$ is a 5-methyl group, $R_2$ and $R_3$ are hydrogen and $R_4$ is a 3'-pyridazinyl radical, in which $R_5$ is a 4',5'-benzo group (PH 22-16),

or R, is a 4-acetamino group, $R_2$ and $R_3$ are hydrogen and $R_4$ is a 3'-pyridazinyl radical, in which $R_5$ is a 4',5'-benzo group (PH 22-18),

or R, is a 4-acetamino group, $R_2$ is a methyl group, $R_3$ is hydrogen and $R_4$ is a 3'-pyridazinyl radical, in which $R_5$ is the 4',5'-benzo group (PH 22-20),

or $R_1$ and $R_3$ are hydrogen, $R_2$ is a phenyl radical and $R_4$ is a 3'-pyridazinyl radical, in which $R_5$ is a 4',5'-benzo group (PH 22-22),

or $R_1$ is a 4-amino group, $R_2$ and $R_3$ are hydrogen, and $R_4$ is a 3'-pyridazinyl radical, in which $R_5$ is a 4' ,5'-benzo group (22-23),

or $R_1$ is a 4-amino group, $R_2$ is a methyl group, $R_3$ is hydrogen and $R_4$ is a 3'-pyridazinyl radical, in which $R_5$ is a 4',5'-benzo group (PH 22-24),

or $R_1$ and $R_3$ are hydrogen, $R_2$ is an ethyl group and $R_4$ is a 3'-pyridazinyl radical, in which $R_5$ is a 4',5'-benzo group (PH 22-25),

or $R_1$, $R_2$, $R_3$ are hydrogen and $R_4$ is a 6'-pyrimidinyl radical, in which $R_5$ is 4'-chlorine (PH 22-26),

or R, $R_2$ $R_3$ are hydrogen and $R_4$ is a 3'-pyridazinyl radical, in which $R_5$ is a 6'-(N)-methyl-(N)-2-hydroxypropylamino group (PH 22-28),

or $R_1$ and $R_3$ are hydrogen, $R_2$ is a methyl group and $R_4$ is a 6'-pyrimidinyl radical, in which $R_5$ is 4'-chlorine (PH 22-32),

or R, is a 5-ethyl group, $R_2$ and $R_3$ are hydrogen and $R_4$ is a 6'-pyrimidinyl radical, in which $R_5$ is a 4'-methoxy group (PH 22-33),

or $R_1$ and $R_3$ are hydrogen, $R_2$ is a methyl group and $R_4$ is a 6'-pyrimidinyl radical, in which $R_5$ is a 4',5'-benzo group (PH 22-34),

or $R_1$ is hydrogen, $R_2$ and $R_3$ are a methyl group and $R_4$ is a 6'-pyrimidinyl radical, in which $R_5$ is 4'-chlorine (PH 22-35),

or $R_1$ and $R_2$ are hydrogen, $R_3$ is a methyl group and $R_4$ is a 6'-pyrimidinyl radical, in which $R_5$ is 4'-chlorine (PH 22-36).

**7.** Drug according to claims 1 to 6 for the treatment of microbial, particularly mycobacterial diseases or malaria or malignant tumours.

**8.** Drug according to claims 1 to 7, characterised in that it contains in addition one or several inhibitors of folate synthase and/or dihydrofolic acid reductase and/or DNA synthesis and/or RNA synthesis.

**9.** Drug according to claim 8, characterised in that it contains as inhibitor of folate synthase a sulphone or a sulphonamide.

**10.** Drug according to claim 8, characterised in that it contains as inhibitor of dihydrofolic acid reductase methotrexate, Brodimoprim, 2,4-diamino-5-{4-[2-(4'aminophenyl-4-sulphonylphenylamino)ethoxy]3,5-dimethoxybenzyl}pyrimidine or 2,4-diamino-5-{4-[2-(4'-aminophenyl-4-sulphonylphenylamino)propoxy]-3,5-dimethoxybenzyl}pyrimidine.

**11.** Drug according to claim 8, characterised in that it contains as inhibitor of DNA synthesis 5-F-Uracil, guanazole or Desferal.

12. Drug according to claim 8, characterised in that it contains as inhibitor of RNA synthesis rifampicin or derivatives thereof.

**Revendications**

1. Médicament ayant une teneur en 2-acylpyridin-α-(N)-hétarylhydrazones substituées de la formule générale I

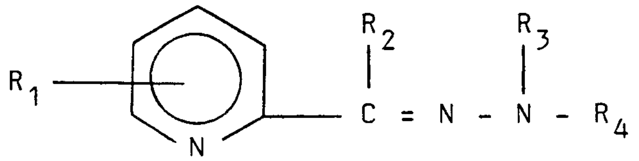

I

où $R_1$ est hydrogène, halogène, un groupe alkyle de 1 à 4 atomes de carbone, un groupe benzyloxy, benzo, amino ou acétamido,
$R_2$ est hydrogène, un groupe alkyle ayant 1 à 8 atomes de carbone, une amine ou un groupe phényle,
$R_3$ est hydrogène ou un groupe méthyle et
$R_4$ est un résidu de formules générales II ou III

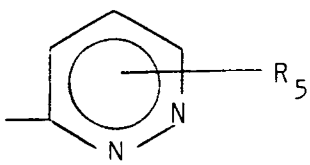

II

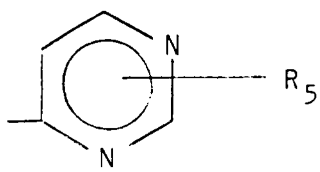

III

où
$R_5$ est hydrogène, halogène, un groupe alkyle ou alcoxy de 1 à 4 atomes de carbone, un groupe benzène ou bien un groupe dialkylamino ou dialkylamino substitué de 1 à 4 atomes de carbone dans les résidus alkyle, en tant qu'agent thérapeutique.

2. Médicament selon la revendication 1, caractérisé en ce qu'il contient, en tant qu'agent thérapeutique,un composé dans lequel $R_1$ et $R_2$ sont indépendamment l'un de l'autre des groupes amine, de l hydrogène ou bien des groupes alkyle de 1 à 4 atomes de carbone.

3. Médicament selon les revendications 1 ou 2, caractérisé en ce qu'il contient, en tant qu'agent thérapeutique,un composé dans lequel $R_1$ est un groupe benzyloxy, benzo ou acétamino.

4. Médicament selon l'une des revendications 1 à 3, caractérisé en ce qu'il contient,en tant qu'agent thérapeutique,un composé dans lequel $R_2$ est un groupe phényle.

5. Médicament selon les revendications 1 à 4, caractérisé en ce qu'il contient, en tant qu'agent thérapeutique un composé dans lequel $R_5$ est un groupe (N)-méthyl-(N)-2-hydroxypropylamino-, méthyl-, chloro-, méthoxy- ou benzène.

6. Médicament selon la revendication 1, caractérisé en ce qu'il contient,en tant qu'agent thérapeutique l'un des composés suivants, dans lesquels :
$R_1$ $R_2$ et $R_3$ sont hydrogène et $R_4$ est un résidu 3'-pyridazinyle, où $R_5$ est un groupe 4',5'-benzène (PH 22-1),
ou bien $R_1$ et $R_3$ sont de l'hydrogène, $R_2$ est un groupe méthyle et $R_4$ est un résidu 3'-pyridazinyle, où $R_5$ est un groupe 4',5'-benzène (PH 22-3),
ou bien $R_1$ est un groupe 5-éthyle, $R_2$ et $R_3$ sont hydrogène et $R_4$ est un résidu 3'-pyridazinyle, où $R_5$ est un groupe 4',5'-benzène (PH 22-5),

ou bien $R_1$ est un groupe 5-benzyloxy, $R_2$ et $R_3$ sont hydrogène et $R_4$ est un groupe 3'-pyridazinyle, où $R_5$ est un groupe 4',5'-benzène (PH 22-6),
ou bien $R_3$ est un groupe 4-méthyle, $R_2$ et $R_3$ sont de l'hydrogène et $R_4$ est un résidu 3'-pyridazinyle, où $R_5$ est le groupe 4',5'-benzène (PH 22-8),
ou bien $R_1$ est un groupe 5-méthyle, $R_2$ et $R_3$ sont de l'hydrogène et $R_4$ est un résidu 3'-pyridazinyle, où $R_5$ est un groupe 4',5'-benzène (PH 22-16),
ou bien $R_1$ et $R_2$ sont de l'hydrogène, $R_3$ est un groupe méthyle et $R_4$ est un résidu 6'-pyrimidinyle, où $R_5$ est 4'-chlore (PH 22-36).

**7.** Médicament selon les revendications 1 à 6, pour le traitement de maladies microbiennes, en particulier mycobactériennes ou bien de la malaria ou de tumeurs malignes.

**8.** Médicament selon les revendications 1 à 7, caractérisé en ce qu'il contient, en plus un ou plusieurs inhibiteurs de la folate synthase et/ou de la dinydrofolate réductase,et/ou de la synthèse de l'ADN et/ou de la synthèse de l'ARN.

**9.** Médicament selon la revendication 8, caractérisé en ce qu'il contient, en tant qu'inhibiteur de la folate syntnase, une sulfone ou bien un sulfonamide.

**10.** Médicament selon la revendication 8, caractérisé en ce qu'il contient en tant qu'inhibiteur de la dihydrofolate réductase, du méthotrexat, du brodimoprim , de la 2,4-diamino-5-{4-[2-(4' aminophényl-4-sulfonyl-phénylamino)éthoxy] 3,5-diméthoxybenzyl} pyrimidine , ou bien de la 2,4-diamino-5-{4-[2-(4'-aminophényl-4-sulfonylphénylamino)-propoxy]3,5-diméthoxybenzyl}pyrimidine.

**11.** Médicament selon la revendication 8, caractérisé en ce qu'il contient, en tant qu'inhibiteur de la synthèse de l'ADN, 5-F-uracil, du guanazol ou du desféral.

**12.** Médicament selon la revendication 8, caractérisé en ce qu'il contient, en tant qu'ihnibiteur de la synthèse de l'ARN, de la Rifampicine ou bien son dérivé.